# DEMANDE DE BREVET EUROPEEN

(11) **EP 0 740 958 A1**
(43) Date de publication de la demande: **06.11.1996**
(21) Numéro de dépôt: 96106412.8
(22) Date de dépôt: 24.04.1996
(51) Int. Cl.: B01L 11/00, G01N 27/28, B65D 83/08

(54) **Appareil pour distribuer des zones successives d'une bande consommable**

(30) Priorité: 02.05.1995 FR 9505223
(71) Demandeur: ASULAB S.A., CH-2501 Bienne (CH)
(72) Inventeur: Jaeger,Gérard, 1807 Blonay (CH)
(74) Mandataire: Patry, Didier Marcel Pierre

(57) **Abrégé**

Cet appareil fonctionne avec des bandes consommables qui y sont introduites par un utilisateur.

Il comprend un couloir (29) pour les bandes (34) dans lequel peut circuler un curseur (35) auquel chaque bande peut être accouplée.

Des moyens sont prévus pour que le curseur (35) ne puisse circuler dans le couloir (29) sans qu'il soit solidaire d'une bande. Par ailleurs, en l'absence de bande (34), le curseur (35) reste bloqué à l'entrée (28) du couloir de circulation.

Application notamment à des dispositifs de distribution d'objets tels que des cachets de médicaments ou à des dispositifs de mesure du taux de glucose dans le sang.

## Description

La présente invention est relative à un appareil distributeur nécessitant pour fonctionner l'emploi d'éléments consommables se présentant sous la forme de bande, chacune de ces bandes comprenant dans le sens longitudinal plusieurs zones d'utilisation successives destinées à être séparées de la bande dès après leur utilisation.

Plus particulièrement, l'invention concerne un tel appareil formant un dispositif de mesure permettant de mesurer un paramètre d'une substance déposée sur des zones successives d'une bande formant capteur de mesure consommable, appelé aussi capteur multizone.

Un tel dispositif de mesure peut avantageusement servir à la mesure du taux de glucose dans le sang, à l'usage des diabétiques. Il a été décrit sous différents aspects dans plusieurs demandes de brevet français déposées au nom de la Demanderesse et parmi lesquelles l'on peut citer le FR 92 01 331 pour ce qui concerne le procédé de mesure électrochimique utilisé, le FR 93 11 316 pour ce qui concerne un dispositif de coupe permettant de séparer de la bande consommable les zones de mesure utilisées, le FR 93 11 317 pour ce qui concerne un dispositif de connexion électrique permettant de relier la bande captrice à un circuit électronique de mesure destiné à élaborer le résultat de la mesure sous une forme perceptible à un utilisateur, et enfin le FR 93 11 319 qui concerne plus spécifiquement un dispositif d'éjection du dernier tronçon de la bande captrice, lorsque toutes les zones de mesure ont été utilisées.

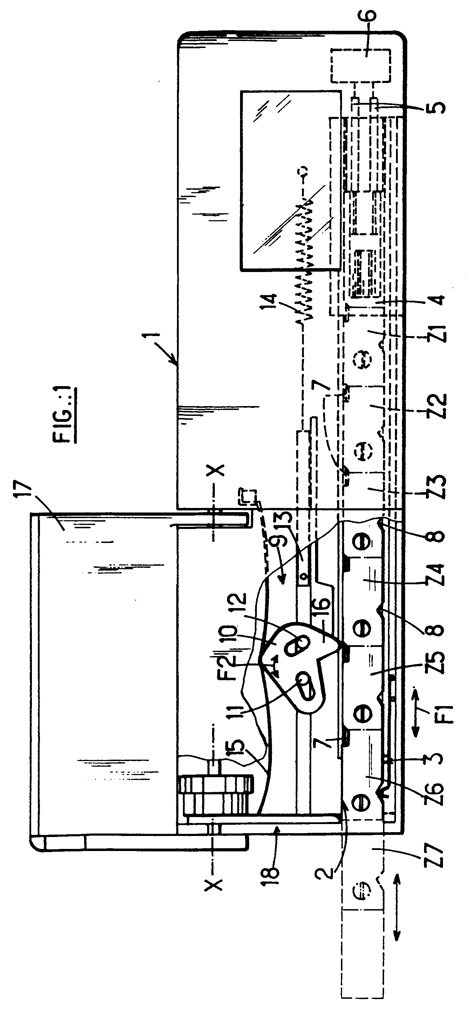

La figure 1 des dessins annexés montre un dispositif de mesure dans lequel on retrouve, sous une forme sommaire, un exemple de réalisation des perfectionnements qui ont fait l'objet des demandes de brevet susindiquées, étant entendu que pour en trouver une description détaillée, l'on peut se référer aux textes desdites demandes de brevet.

Ainsi, le dispositif de mesure comporte un boîtier 1, de forme générale allongée et d'une taille telle qu'il puisse facilement être tenu dans le creux de la main d'un adulte. Ce boîtier définit un couloir 2 de circulation orienté longitudinalement dans le boîtier 1 et destiné à la circulation d'une bande captrice 3 (double flèche F1) ainsi que d'un curseur 4. Ce dernier est chargé de transmettre le signal électrique provenant de la bande captrice 3 à des lignes électriques 5 s'étendant le long du couloir 2. Ces lignes sont à leur tour connectées à un circuit électronique 6 destiné à exploiter convenablement ce signal électrique pour le rendre intelligible à un utilisateur au moyen d'un dispositif d'affichage prévu également dans le boîtier 1.

Dans l'exemple de la figure 1, la bande captrice 3 est conformée spécifiquement à la mesure du taux de glucose dans le sang. Une description détaillée d'une telle bande peut être trouvée dans la première demande de brevet précitée. Il suffit de retenir ici qu'elle comporte plusieurs zones de mesure, sept en l'occurrence référencées Z1 à Z7 dont la zone Z7 représentée en pointillés est supposée avoir déjà été utilisée et tronçonnée de la bande 3.

On remarquera également que la bande captrice comprend pour toutes les zones Z1 à Z7 un cran d'avancement 7 sur l'un de ses bords longitudinaux ainsi que pour toutes les zones sauf la zone Z7 une encoche de positionnement 8 située sur l'autre bord de la bande.

Les crans d'avancement 7 coopèrent avec un mécanisme 9 d'avance longitudinal de la bande 3 le long de son couloir 2. Ce dernier comprend un bouton d'actionnement (non visible sur la figure 1) monté en coulissement longitudinal dans le boîtier c'est-à-dire comme vu sur la figure 1, au dessus du couloir 2.

Le bouton d'actionnement coopère avec un cliquet basculant 10 guidé sur deux tétons 11 et 12 solidaires d'une réglette 13. Celle-ci est couplée au bouton d'actionnement et montée coulissante dans le boîtier 1 dans la direction de la flèche F1 à l'encontre de l'action d'un ressort de rappel 14 ancré sur le boitier 1. Le cliquet basculant 10 est maintenu en position non active (représentée sur la figure 1) par un ressort à lame 15 et comporte un bec d'actionnement 16 destiné à coopérer avec les crans d'avancement 7 de la bande captrice 3.

Dès lors, on comprend que le bouton d'actionnement étant activé en va et vient, la bande captrice avance de la longueur d'une zone de mesure de la bande, le mécanisme d'avance faisant basculer le cliquet 10 en va et vient (selon la flèche F2) pour d'abord pousser la bande hors du couloir d'un pas puis revenir vers la position inactive telle que représentée en se dégageant de la bande.

Le dispositif comprend aussi un couvercle 17 monté rotatif sur le boîtier autour d'un axe X-X et couplé mécaniquement à un mécanisme de coupe 18 permettant de tronçonner la zone de la bande consommable que l'on vient d'utiliser, et ce par un simple mouvement de fermeture du couvercle 17.

Il est encore à noter que le curseur 4 coulisse librement dans le couloir 2 et est repoussé vers l'arrière lors de l'insertion de la bande captrice consommable 3. Celle-ci y est couplée mécaniquement lors de cette insertion lorsque le curseur 4 vient buter contre le fond du couloir 2. A cet effet, le curseur porte un organe de couplage élastique cédant sous la force d'insertion exercée sur la bande 3 par l'utilisateur, pour s'accrocher à cette bande dès que la force d'insertion est relâchée.

Des tests d'utilisation du dispositif de mesure qui vient d'être décrit, ont montré qu'il fonctionne convenablement et sans difficultés tant que les bandes sont consommées entièrement et ont une longueur initiale suffisante pour pouvoir être couplées au curseur, lorsque ce dernier est situé au fond du couloir.

De ce fait, des bandes partiellement consommées ou ayant une longueur plus faible à la fabrication ne peuvent être utilisés, étant donné que, dans ce cas, le couplage avec le curseur ne peut être effectué.

L'invention a pour but d'apporter une solution au problème évoqué ci-dessus.

L'invention a donc pour objet un appareil distributeur nécessitant pour fonctionner l'emploi d'éléments consommables se présentant sous la forme de bandes, chacune de ces bandes comprenant dans le sens longitudinal plusieurs zones d'utilisation successives destinées à être séparées de la bande dès après leur utilisation, ledit appareil comprenant:
- un boîtier définissant un couloir de circulation pour lesdites bandes, ledit couloir présentant, compte tenu du sens d'introduction desdites bandes, une extrémité amont et une extrémité aval,
- un mécanisme d'avance pour faire sortir ladite bande du couloir de circulation selon un mouvement pas-à-pas après son introduction dans celui-ci par un utilisateur,
- une unité coulissante montée mobile dans ledit couloir, couplée fonctionnellement audit mécanisme d'avance et destinée à être accouplée à l'extrémité correspondante de ladite bande lors de son introduction par l'utilisateur, et
- des moyens de couplage portés par ladite unité coulissante et agencés pour assurer le couplage entre celle-ci et ladite bande,
   ledit appareil étant caractérisé en ce que ledit boîtier définit également des moyens formant butée situés à l'extrémité amont dudit couloir, et en ce qu'il comprend en outre des moyens de blocage agencés pour, en coopération avec lesdits moyens formant butée, assurer le blocage de ladite unité coulissante dans une position située en amont dudit couloir en l'absence d'une bande dans celui-ci et pour libérer ladite unité coulissante a se mouvoir dans ledit couloir dès le couplage de l'extrémité aval de ladite bande avec cette unité coulissante.

Grâce à ces caractéristiques, l'unité coulissante sera bloquée à l'embouchure amont du couloir de circulation, position dans laquelle elle pourra toujours être ramenée par le mécanisme d'avance et à partir de laquelle elle ne pourra se déplacer vers l'aval du couloir que lorsqu'elle est accouplée à une bande consommable.

D'autres caractéristiques et avantages de l'invention apparaîtront au cours de la description qui va suivre, donnée uniquement à titre d'exemple et faite en se référant aux dessins annexés sur lesquels:
- la figure 1, déjà décrite, est une vue schématique en plan, avec arrachement partiel, d'un appareil distributeur conforme aux demandes de brevet précitées;
- les figures 2A, 2B et 2C sont trois vues extérieures, selon trois plans orthogonaux, d'un appareil distributeur selon l'invention;
- la figure 3 est une vue en plan d'un appareil distributeur selon l'invention, la partie supérieure de son boîtier étant supposée enlevée;
- la figure 4 est une vue en plan, à échelle agrandie, du curseur de l'appareil selon l'invention, pour en illustrer les caractéristiques essentielles;
- la figure 5 est une vue en coupe prise selon la ligne brisée V-V de la figure 4;
- la figure 6 est une vue en bout du couloir de guidage des bandes consommables.
- la figure 7 est une vue en bout d'un curseur destiné à se déplacer dans le couloir de guidage;
- la figure 8 montre en plan le début du couloir de guidage ainsi que des moyens formant came et butée qui y sont ménagés;
- la figure 9 montre une vue en perspective de moyens élastiques de blocage et de couplage faisant partie de l'appareil selon l'invention; et
- les figures 10A, 10B et 10C sont des vues schématiques analogues à la figure 4 et représentant schématiquement le fonctionnement de l'appareil selon l'invention.

Les figures représentent un exemple préféré de réalisation de l'appareil distributeur suivant l'invention dans son application à un dispositif permettant de mesurer le taux de glucose dans le sang à l'usage des diabétiques, par exemple. Cependant, l'invention n'est pas limitée à cette application spécifique. En effet, elle peut être mise en oeuvre, dans toute sorte d'autres cas de figure dans lesquels il est souhaitable d'utiliser des bandes consommables sur lesquelles des zones adjacentes définissent une aire d'utilisation spécifique, ces zones, après avoir été utilisées pouvant, voire devant (pour des raisons médicales par exemple) être ôtées de la bande pour laisser la place à une zone suivante. Par exemple, dans le domaine médicale, un tel appareil pourrait être utilisé pour la distribution d'une série de cachets ou pilules de médicaments devant, selon la posologie prescrite par le médecin, être pris dans un ordre strict défini à l'avance. Toutefois, l'invention ne se limite pas davantage aux applications dans le domaine médical.

L'appareil distributeur selon l'invention représenté aux figures comporte un boîtier 20 fait en deux demicoquilles 20A et 20B, en matière plastique moulée de préférence, et assemblées l'une à l'autre selon un plan de joint 21. Dans l'application décrite, le boîtier 20 a une forme allongée et présente des dimensions telles qu'il puisse facilement être tenu dans le creux de la main d'un adulte.

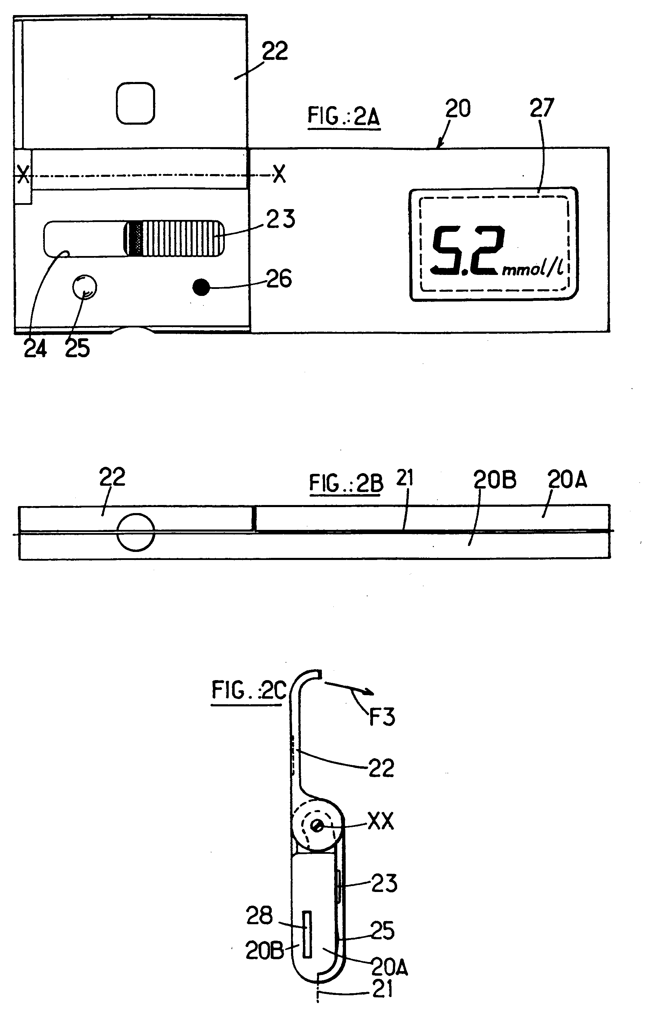

L'appareil comporte un couvercle 22 s'étendant sur une partie de sa longueur et articulé sur le boîtier 20 autour d'un axe longitudinal X-X et qui peut être rabattu sur le boîtier 20 en l'actionnant dans le sens de la flèche F3, (figure 2C).

Sur les figures 2A et 2C, on a représenté le couvercle 22 en position ouverte, de sorte que l'on aperçoit un bouton 23 coulissant dans un guide 24 ménagé dans la demi-coquille supérieure 20A du boîtier 20. Une petite loupe 25 permet d'observer une partie de la bande consommable (non représentée ici) glissée dans l'appareil. Un poussoir 26 est destiné à rappeler des mesures effectuées antérieurement et stockées dans la mémoire de l'appareil, pour l'affichage sur un écran 27 servant à permettre également la lecture immédiate des résultats de la mesure qui vient d'être faite.

Il est à noter que l'appareil est mis en état de marche, dès que le bouton 23 est actionné et que son alimentation (par une pile incorporée de préférence) est coupée après l'écoulement d'un délai prédéterminé (60 secondes, par exemple) défini à partir du moment où toute intervention sur l'appareil par l'utilisateur a cessé.

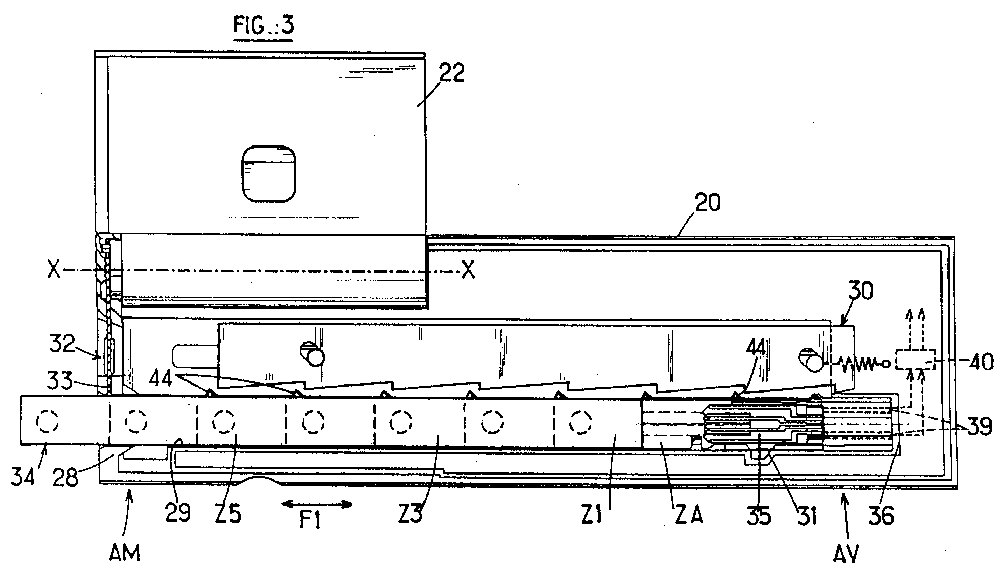

Du côté du couvercle 22, sur le chant latéral du boîtier 20, l'appareil présente une ouverture 28 qui est l'embouchure d'un couloir de circulation 29 (figure 3) des bandes consommables.

La figure 3 montre l'appareil distributeur selon l'invention, alors que l'on en a ôté la demi-coquille supérieure 20A. De la sorte, on peut observer plusieurs unités fonctionnelles et notamment:
- un mécanisme d'avance 30 qui ne sera pas décrit en détail ici, mais au sujet duquel il suffit de retenir qu'il permet d'avancer pas-à-pas une bande consommable dans le couloir 29 sous l'action répétée du bouton coulissant 23;
- une unité coulissante 31, destinée à être accouplée à une bande consommable et coulissant dans le couloir 29;
- une unité de coupe 32 située le long du chant latéral de l'appareil et comprenant un couteau 33 monté coulissant dans une direction perpendiculaire à l'axe X-X; et
- une bande consommable 34 présentant sept zones de mesure Z1 à Z7 et une zone ZA formant amorce et permettant son couplage à l'unité coulissante 31.

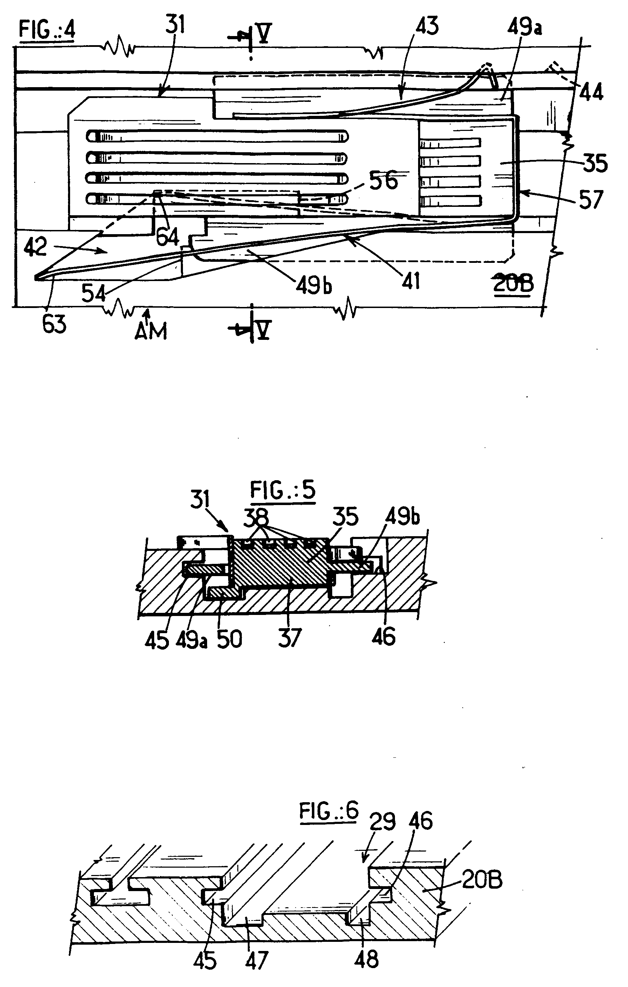

On va maintenant se référer aux figures 4 à 9 pour décrire plus spécifiquement les caractéristiques de la présente invention qui ont trait à l'unité coulissante 31 et la façon de coupler celle-ci à une bande consommable 34.

L'unité coulissante 31 comprend un curseur 35 pouvant circuler dans le couloir 29 qui est obturé à son extrémité 36 opposée à l'embouchure 28. Dans ce qui va suivre, on appellera cette extrémité, "extrémité aval" AV en considérant le mouvement d'introduction de la bande consommable 34 dans le couloir 29. Bien entendu, l'extrémité opposée, près de l'embouchure 28 sera appelée "extrémité amont" AM. Ces termes seront de même utilisés pour la bande consommable, son extrémité aval étant donc située près de la zone d'amorce ZA.

Le curseur 35 comprend un corps de curseur 37 portant des moyens électriques de transmission 38 chargés d'assurer le prélèvement des signaux électriques de la bande 34 et la transmission de ces signaux à des pistes de contact 39 qui courent le long de la paroi supérieure du couloir 29 (comme vu sur les figures). Selon une variante, on peut également prévoir à cet effet un câble flexible plat qui se développe dans le couloir 29.

Les pistes 39 sont connectées à un circuit électronique de traitement 40 relié à son tour à l'écran d'affichage 27 (figure 2A).

Le corps 37 porte également des moyens élastiques de blocage et de couplage 41 qui sont conçus conformément aux caractéristiques de la présente invention. Ces moyens 41 coopèrent avec des moyens 42 formant came et butée qui sont venus de moulage dans la demi-coquille inférieure 20B du boîtier 20.

Le corps 37 porte en outre des moyens 43 d'entraînement et de positionnement destinés à coopérer avec les moyens d'avance 30 et avec des crans d'indexage 44 prévus le long du couloir 29 et définissant respectivement les positions de mesure de la bande 34.

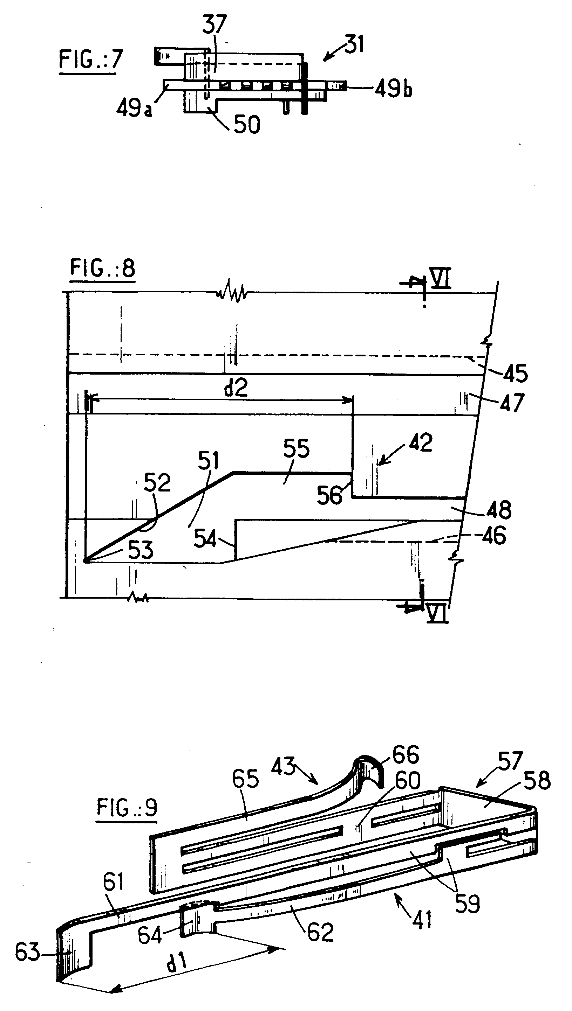

Sur les figures 6 et 8, on voit que le couloir de guidage 29 présente deux rainures longitudinales latérales 45, 46 et deux rainures longitudinales 47 et 48 ménagées dans son fond. Le corps 37 (figure 7) est de forme générale parallélépipédique d'où font saillie deux nervures latérales 49a et 49b, s'ajustant respectivement dans les rainures latérales 45 et 46 et une nervure inférieure 50 qui s'ajuste dans la rainure 47 prévue dans le fond du couloir 29.

La figure 8 montre les moyens 42 formant came et butée qui sont ménagés par moulage dans la demi-coquille inférieure 20B. Plus précisément, près de l'embouchure 28, la paroi extérieure de la rainure 48 présente une encoche 51 dont la paroi amont 52 (vu dans le sens de l'introduction de la bande 34) est placée de biais. Cette paroi fait office de came et la pointe 53 ainsi ménagée dans cette encoche 51 constitue une butée. L'encoche 51 définit également une butée aval 54 orientée perpendiculairement à la direction longitudinale de la rainure 48.

En face, la paroi latérale intérieure de la rainure 48 présente également une encoche 55 dont la paroi amont prolonge de biais la paroi 52 et dont la paroi aval est également orientée perpendiculairement à la rainure 48 pour former une butée 56.

Les moyens élastiques 41 de blocage et de couplage et les moyens 43 d'entraînement et de positionnement sont de préférence réalisés dans une même lame élastique 57 (figure 4 et 9). Celle-ci présente une forme en U et peut par exemple être surmoulée par la matière plastique au moment du formage du curseur 35. L'âme 58 du U est appliquée contre la face aval du curseur 35 tandis que ses branches 59 et 60 s'étendent vers l'embouchure 28 du couloir 29 en longeant respectivement les flancs latéraux du curseur 35.

La branche 59 est fendue longitudinalement pour former deux ressorts superposés 61, 62 de longueurs inégales et se terminant chacun à son extrémité libre par une palette 63, 64 recourbée vers l'extérieur. L'autre branche 60 est également fendue longitudinalement pour d'une part permettre l'accrochage de la matière plastique au cours du moulage et d'autre part définir un ressort 65 qui s'étend vers l'aval et se termine par un crochet 66.

L'élasticité des ressorts 61, 62 et 65 est déterminée de telle façon que celle des ressorts 61 et 62 sollicite leur palette respective 63, 64 vers l'intérieur, tandis que celle du ressort 65 sollicite le crochet 66 vers l'extérieur.

On voit en particulier sur les figures 3 et 10A à 10C que la zone d'amorce ZA de la bande consommable présente à son extrémité avant, d'une part un biseau 67 formant came et, d'autre part située légèrement vers l'arrière une encoche latérale de couplage 68. On remarquera qu'ainsi la bande consommable est dissymétrique vis-a-vis de son axe longitudinal de sorte que le biseau en particulier forme détrompeur qui ne laisse à l'utilisateur qu'une seule façon pour l'introduire dans l'appareil.

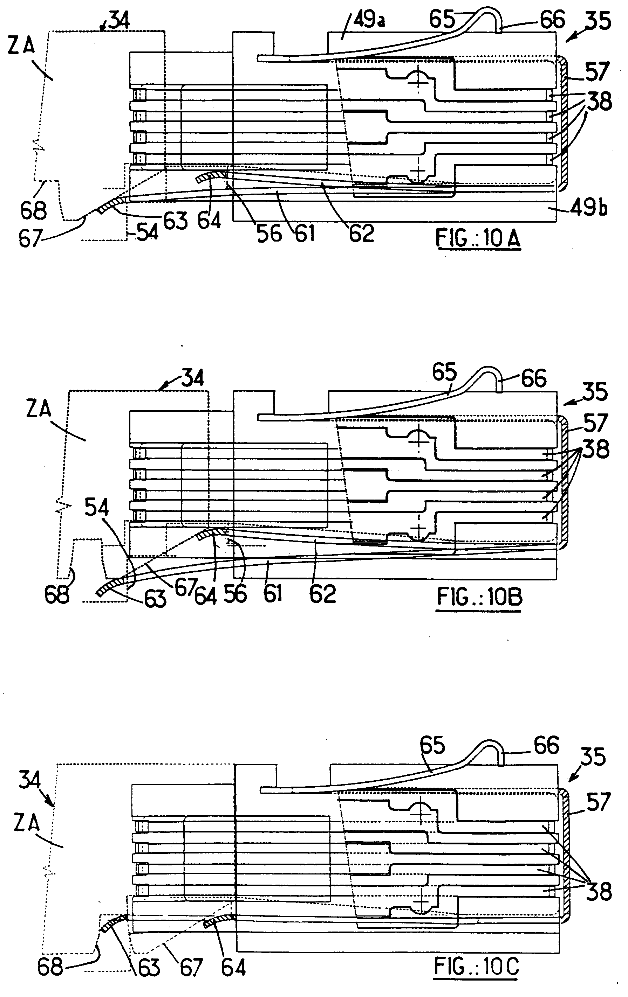

Les figures 4, 10A, 10B et 10C montrent diverses positions des organes de l'appareil, respectivement avant et pendant l'introduction d'une bande consommable 34 dans l'appareil.

Tout d'abord sur la figure 4, la zone d'amorce ZA d'une bande consommable précédente a été éjectée du couloir 29 et, ayant été amené vers l'amont lors de l'utilisation de cette bande, le curseur 35 se trouve en amont dans le couloir 29. Dans cette position, ce curseur est empêché de se déplacer vers l'aval et, bien entendu, aussi de sortir du couloir 29. En effet, la palette 64 est en position dans l'encoche 55 du boîtier, la butée 56 s'opposant à tout mouvement du curseur vers l'aval. Par ailleurs, la palette 63 du ressort 61 se trouve repoussée vers l'extérieur sur la rampe 52, la pointe ou butée 53 s'opposant à tout mouvement vers l'amont.

Il convient de noter que dans cette configuration, le curseur 35 conserve de préférence une certaine liberté de mouvement dans le couloir 29, la distance d1 entre le bord amont actif 63a (figure 9) de la palette 63 et le bord aval actif 64a de la palette 64 étant légèrement inférieure à la distance d2 (figure 8) qui sépare la butée 53 de la butée 56.

Sur la figure 10A, la bande consommable 34 vient d'être présentée devant l'embouchure 28 (figure 3) et sa zone d'amorce ZA y est déjà introduite sur une petite distance. En poussant sur la bande, l'utilisateur va alors provoquer le recul du curseur 35 par la mise en contact de la rampe 67 avec la palette 63. Ce mouvement se poursuit jusqu'à ce que la palette vienne en appui contre la butée 56.

L'utilisateur en continuant à exercer une force d'introduction sur la bande 34, va vaincre la résistance des moyens électriques de transmission 38 qui jusqu'alors résistaient à leur propre déformation élastique. La bande peut donc continuer à s'introduire et s'accoupler au curseur 35, tandis que la 67 rampe commence a repousser vers l'extérieur la palette 64 du ressort 62 (figure 10B). A peu près au même moment, la palette 63 a atteint la fin de la rampe 67 de la zone d'amorce ZA,
Dès que la palette 64 se dégage de la butée 56, elle peut désormais s engager vers l'aval dans le couloir 29. Il est à noter que la palette 64 doit se dégager de la butée 56 avant que la palette 63 ne tombe dans l'encoche 68.

Cependant, le curseur 35 ne peut pas partir tant que la palette 63 reste en appui contre le bord latéral de la zone d'amorce ZA, compte tenu de la présence de la butée 54. Mais, dès que la palette 63 peut s'introduire dans l'encoche 68 de la zone d'amorce ZA, le curseur peut se déplacer poussé par la bande 34. En effet, l'encoche 68 est assez profonde pour que la palette 63 se place à la hauteur de la rainure 48 du couloir 29.

Par conséquent, comme représenté sur la figure 10C, les deux palettes se trouvent alors dans une position dans laquelle elles peuvent circuler dans la rainure 48, ce dont il résulte que le curseur 35 est désormais libre de se déplacer vers l'aval. La bande consommable 34 lui est solidaire tant que le curseur n'est pas ramené de nouveau vers l'amont par l'intermédiaire du mécanisme d'avance 31.

On voit donc que les deux ressorts 61 et 62 forment des organes élastiques qui, lors de l'introduction de la bande consommable 34, se relayent pour bloquer provisoirement le mouvement du curseur 35 dans le couloir 29 tant que l'accrochage mutuel du curseur et de la bande consommable ne se soit pas produit. De ce fait, le curseur 35 ne peut pas se déplacer vers l'arrière sans être accouplé à la bande consommable. En d'autres termes, le curseur ne peut se trouver que dans la position amont de la Figure 10A, lorsqu'il n'y a pas de bande consommable ou de partie de celle-ci dans l'appareil.

Il est à noter par ailleurs que les organes élastiques ou ressorts 61 et 62 libèrent la bande consommable dès que le curseur se retrouve dans la position amont, car dans ces conditions, la palette 63 est repoussée hors de l'encoche 68 de la bande consommable.

## Revendications

1. Appareil distributeur nécessitant pour fonctionner l'emploi d'éléments consommables se présentant sous la forme de bande (34), chacune de ces bandes comprenant dans le sens longitudinal plusieurs zones d'utilisation successives (Z1 à Z7) destinées à être séparées de la bande dès après leur utilisation, ledit appareil comprenant:
- un boîtier (20) définissant un couloir de circulation (29) pour lesdites bandes, ledit couloir présentant, compte tenu du sens d'introduction desdites bandes, une extrémité amont (AM) et une extrémité aval (AV),
- un mécanisme d'avance (30) pour faire sortir ladite bande (34) du couloir de circulation (29) selon un mouvement pas-à-pas (F1) après son introduction dans celui-ci par un utilisateur,
- une unité coulissante (31) montée mobile dans ledit couloir (29), couplée fonctionnellement audit mécanisme d'avance (30) et destinée à être accouplée à l'extrémité correspondante de ladite bande (34) lors de son introduction par l'utilisateur, et
- des moyens de couplage (41) portés par ladite unité coulissante (31) et agencés pour assurer le couplage entre celle-ci et ladite bande (34),
ledit appareil étant caractérisé en ce que ledit boîtier (20) définit également des moyens formant butée (42) situés à l'extrémité amont (AM) dudit couloir (29), et en ce qu'il comprend en outre des moyens de blocage (41) agencés pour, en coopération avec lesdits moyens formant butée (42), assurer le blocage de ladite unité coulissante (31) dans une position située en amont (AM) dudit couloir (29) en l'absence d'une bande (34) dans celui-ci et pour libérer ladite unité coulissante (31) à se mouvoir dans ledit couloir (29) dès le couplage de l'extrémité aval (ZA) de ladite bande (34) avec cette unité coulissante.

2. Appareil suivant la revendication 1, caractérisé en ce que lesdits moyens de blocage (41) sont agencés pour n'autoriser la libération de ladite unité coulissante (31) que lorsque le couplage entre ladite bande (34) et cette unité est achevé.

3. Appareil suivant la revendication 2, caractérisé:
- en ce que lesdits moyens de blocage (41) comprennent des premier et second organes élastiques (61, 62);
- en ce que lesdits moyens formant butée (42) comprennent des première et seconde butées (54, 56) destinées à coopérer respectivement avec lesdits premier et second organes élastiques (61, 62) de manière à s'opposer au mouvement de ladite unité coulissante (31) de l'extrémité amont (AM) vers l'extrémité aval (AV), tant que ledit couplage entre ladite bande et ladite unité coulissante n'est pas achevé;
- en ce que ledit premier organe élastique (61) définit également un organe de couplage desdits moyens de couplage destiné à venir en prise avec ladite bande (34) lors du couplage de celle-ci avec ladite unité coulissante (31); et
- en ce que, lors de l'introduction de ladite bande (34), ledit premier organe élastique (61) ne peut s'effacer devant ladite première butée (54) pour se coupler à ladite bande, avant que le second organe élastique (62) soit dégagé de ladite seconde butée (56).

4. Appareil suivant la revendication 3, caractérisé en ce que lesdits organes élastiques (61, 62) sont des ressorts à lame fixés à une extrémité sur ladite unité coulissante (31), s'étendant dans la direction du couloir de circulation (29) et portant à leur autre extrémité une palette (63, 64) destinée à coopérer avec ladite butée correspondante (54, 56).

5. Appareil suivant la revendication 4, caractérisé en ce que les palettes (63, 64) desdits ressorts à lame (61, 62) sont agencées pour coopérer avec un bord en forme de rampe (67) de chacune desdites bandes (34).

6. Appareil suivant l'une quelconque des revendications 4 et 5, caractérisé en ce que la palette (63) dudit premier organe élastique (61) est agencée pour s'engager dans une encoche (68) prévue dans le bord latéral de chacune desdites bandes (34) pour assurer la fixation de la bande à ladite unité coulissante (31).

7. Appareil suivant l'une quelconque des revendications 4 à 6, caractérisé en ce que lesdits moyens formant butée comprennent également une butée supplémentaire (53) ainsi qu'une rampe (52) situées à l'extrémité amont dudit couloir (29) et destinées à coopérer avec ladite palette (63) dudit premier organe élastique (61) pour s'opposer à l'extraction de ladite unité coulissante dudit couloir (29).
